# EUROPÄISCHE PATENTSCHRIFT

(11) **EP 3 037 085 B1**
(45) Veröffentlichungstag und Bekanntmachung des Hinweises auf die Patenterteilung: **03.04.2019**
(21) Anmeldenummer: 16000168.1
(22) Anmeldetag: 27.08.2013
(51) Int. Cl.: A61K 8/39, A61K 8/81, A61Q 17/04, A61K 8/60, A61K 8/06

(54) **KOSMETISCHE EMULSION MIT POLYACRYLATEN**
COSMETIC EMULSION WITH POLYACRYLATES
ÉMULSION COSMETIQUE CONTENANT DES POLYACRYLATES

(30) Priorität: 01.10.2012 DE 102012217894
(43) Veröffentlichungstag der Anmeldung: 29.06.2016
(62) Teilanmeldung aus: 13181888.2
(73) Patentinhaber: Beiersdorf AG, 20253 Hamburg (DE)
(72) Erfinder: Ulrich, Frauke, 22525 Hamburg (DE); Bleckmann, Andreas, 22926 Ahrensburg (DE); Von Davier, Anabelle, 22527 Hamburg (DE)

(56) Entgegenhaltungen:
- EP-A1- 2 105 124
- DE-A1-102006 034 531
- DE-A1-102009 048 977
- DE-U1-202006 011 833
- DATABASE GNPD [Online] MINTEL; 1. Juli 2012 (2012-07-01), Jialan Group: "Aging Resistance Activating Emulsion", XP002740822, Database accession no. 1828429
- DATABASE GNPD [Online] MINTEL; 1. Oktober 2009 (2009-10-01), Torunskie Zaklady: "Body Care Cream", XP002740823, Database accession no. 1165843
- DATABASE GNPD [Online] MINTEL; 1. November 2011 (2011-11-01), Amorepacific: "Skin Renewal Creme", XP002740824, Database accession no. 1641480
- DATABASE GNPD [Online] MINTEL; 1. Juli 2011 (2011-07-01), LG Household & Health Care: "Sooryehan Yunha Whitening - Cream", XP002740825, Database accession no. 1577425
- DATABASE GNPD [Online] MINTEL; 30 September 2017 (2017-09-30), Beiersdorf: "Nivea Intensive Moisture Serum SPF 25 PA ++", Database accession no. 1896919

## Beschreibung

Die vorliegende Erfindung betrifft eine kosmetische Emulsion enthaltend Polyglyceryl-3 Methylglucose Distearate, Natriumpolyacrylat Homopolymer (INCI Sodium Polyacrylate) und quervernetzte Polyacrylsäure und/oder deren Salze.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren haben dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welcne die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Die Vielzahl an kommerziell erhältlichen kosmetischen Emulsionen darf jedoch nicht darüber hinwegtäuschen, dass diese Zubereitungen des Standes der Technik eine Reihe von Nachteilen aufweisen.

Nachteilig am Stande der Technik ist beispielsweise der Umstand, dass herkömmliche Emulsionen mit Polyglyceryl-3 Methylglucose Distearate relativ schwer in die Haut einziehen und zu einem starken, fettigen Hautglanz führen. Die Zubereitungen fühlen sich darüber hinaus relativ ölig an, neigen zur Röllchenbildung beim Verteilen auf der Haut und lassen sich relativ schwer aus Textilien wieder auswaschen, die bei der Applikation und Anwendung der Emulsion mit derselben kontaminiert wurden.

Verwendet man diese Emulsionsgrundlagen als Basis für Sonnenschutzmittel (d.h. setzt man den Zubereitungen UV-Filter zu), so "weißeln" die Zubereitungen relativ stark, ein Effekt, der insbesondere durch die Agglomeration von partikulären UV-Filtern wie Titandioxid ausgelöst wird.

Es war daher die Aufgabe der vorliegenden Erfindung, die Nachteile des Standes der Technik zu beseitigen oder zumindest zu reduzieren.

Überraschend gelöst werden die Aufgaben durch eine kosmetische Emulsion enthaltend
a) Polyglyceryl-3 Methylglucose Distearate,
b) Natriumpolyacrylat Homopolymer (INCI Sodium Polyacrylate) und
c) quervernetzte Polyacrylsäure und/oder deren Salze, dadurch gekennzeichnet, dass die Zubereitung Ethylhexylsalicylat enthält, wobei das Gewichtsverhältnis von Natriumpolyacrylat Homopolymer (INCI Sodiumpolyacrylate) zu quervernetzter Polyacrylsäure und/oder deren Salze von 1:7 bis 1:2 beträgt.

Die erfindungsgemäßen Zubereitungen weisen beim Auftragen auf die Haut eine erstaunlich leichte, pudrige Sensorik auf. Die Röllchenbildung beim Verteilen der Emulsion auf der Haut ist, obwohl mindestens zwei Polymere in der Zubereitung vorhanden sind, deutlich reduziert. Die Zubereitungen ziehen schnell in die Haut ein und wirken auch bei höherem Gehalt an lipophilen Verbindungen nicht ölig. Aus diesem Grunde sind sie besonders geeignet als Grundlage für Sonnenschutzmittel, die einen relativ hohen Anteil an bei Raumtemperatur flüssigen UV-Filtern wie Octocrylen oder Ethylhexylsalicylat enthalten.

Verwendet man diese Emulsionsgrundlagen als Basis für Sonnenschutzmittel (d.h. setzt man den Zubereitungen UV-Filter zu), so "weißeln" die Zubereitungen deutlich weniger und haben darüber hinaus einen höheren Lichtschutzfaktor (SPF) als für die Einsatzkonzentrationen der UV-Filter zu erwarten ist. Durch ihren überraschend gelblicheren Farbton ermöglichen diese Zubereitungen darüber hinaus eine besonders einfache Kontrolle der gleichmäßigen Verteilung der Emulsion auf die Haut. Der Verbraucher kann damit, bevor die Zubereitung in die Haut einzieht, überprüfen, dass die Zubereitung gleichmäßig auf der Haut verteilt ist, das insbesondere bei Sonnenschutzmitteln von Bedeutung ist.

Nicht zuletzt lässt sich die Emulsion deutlich leichter wieder aus Textilien auswaschen, die mit der Zubereitung beim Auftragen oder während der Anwendung in Kontakt gekommen sind.

Zwar kennt der Stand der Technik die DE 20 2006 011 833 und die DE 10 2007 024 345, doch konnten diese Schriften nicht den Weg zu dieser Erfindung weisen. Darüber hinaus kennt der Fachmann die DE 10 2009 048977, DE 10 2006 034 531, EP 2 105 124 und die Database GNPD Mintel Einträge Jialan Group "Aging Resistance Activating Emulsion" (Database accession no. 1828429), Torunskie Zakldy "Body Care Cream"(Database accession no. 1165843), Amorepacific "Skin Renewal Creme" (Database accession no. 1641480) und LG Household & Health Care "Sooryehan Yunha Whitening Cream" (Database accession no. 1577425), Nivea Body "Intensive Moisture Serum SPF 25PA++" (Database accession no. 1896919), die ebenfalls nicht den Weg zur vorliegenden Erfindung weisen konnten.

Der Emulgator Polyglyceryl-3 Methylglucose Distearate kann bei der Firma Evonik unter dem Handelsnamen Tegocare 450 käuflich erworben werden.

Es ist erfindungsgemäß vorteilhaft, wenn die Zubereitung Polyglyceryl-3 Methylglucose Distearate in einer Konzentration von bis Gewichts-% 0,5 bis 4 Gew%, bevorzugt 1,0 bis 3,0 Gew%, besonders bevorzugt 1,5 bis 2,5 Gew%, bezogen auf das Gesamtgewicht der Zubereitung, enthält.
Erfindungsgemäß bevorzugt handelt es sich bei der erfindungsgemäßen Emulsion um eine O/W-Emulsion.

Erfindungsgemäß vorteilhafte Ausführungsformen der vorliegenden Erfindung sind, dadurch gekennzeichnet, dass die Zubereitung Natriumpolyacrylat Homopolymer (INCI Sodium Polyacrylate) in einer Konzentration von bis Gewichts-% 0,01 Gew% bis 1,0 Gew%, bevorzugt 0,05 Gew% bis 0,75 Gew%, besonders bevorzugt 0,1% bis 0,5 Gew%, bezogen auf das Gesamtgewicht der Zubereitung, enthält.

Das erfindungsgemäße Natriumpolyacrylat kann beispielsweise bei der Firma BASF unter dem Handelsnamen Verdemia SP käuflich erworben werden.

Als erfindungsgemäß vorteilhafte quervernetzte Polyacrylsäure werden Carbomere und/oder Acrylates/C10-30 Alkyl Acrylate Crosspolymere eingesetzt. Erfindungsgemäß vorteilhafte Carbomere sind dabei insbesondere die Carbomere 980 und 981. Erfindungsgemäß besonders bevorzugt ist das Carbomer Carbopol 980.

Es ist erfindungsgemäß vorteilhaft, wenn die erfindungsgemäße Zubereitung quervernetzte Polyacrylsäure und/oder deren Salze in einer Konzentration von 0,1 bis 1,0 Gewichts-%, bezogen auf das Gesamtgewicht der Zubereitung, enthält.

Es ist erfindungsgemäß bevorzugt, wenn die erfindungsgemäße Zubereitung quervernetzte Polyacrylsäure und/oder deren Salze in einer Konzentration von 0,2 bis 0,7 Gewichts-%, besonders bevorzugt in einer Konzentration von 0,3 bis 0,6 Gewichts-% bezogen auf das Gesamtgewicht der Zubereitung, enthält.

Insbesondere sind die erfindungsgemäß vorteilhaften Ausführungsformen der vorliegenden Erfindung dadurch gekennzeichnet, dass das Gewichtsverhältnis von Natriumpolyacrylat Homopolymer (INCI Sodiumpolyacrylate) zu quervernetzter Polyacrylsäure und/oder deren Salze besonders bevorzugt von 1:3 bis 1: 6 beträgt.

Eine erfindungsgemäß vorteilhafte Ausführungsform der vorliegenden Erfindung stellen Sonnenschutzmittel dar. Dabei handelt es sich erfindungsgemäß vorteilhaft um Sonnenschutzmittel mit einem hohen Lichtschutzfaktor (SPF), d.h. mit einem Lichtschutzfaktor von mindestens SPF 25.

Derartige erfindungsgemäße Zubereitungen sind erfindungsgemäß vorteilhaft dadurch gekennzeichnet, dass die Zubereitung einen oder mehrere UV-A-Filter gewählt aus der Gruppe der Verbindungen 4-(tert.-Butyl)-4'-methoxydibenzoylmethan und 2-(4'-Diethylamino-2'-hydoxybenzoyl)-benzoesäurehexylester enthält.

Dabei ist es erfindungsgemäß bevorzugt, wenn der UV-A-Filter Verbindungen 4-(tert.-Butyl)-4'-methoxydibenzoylmethan eingesetzt wird.

Die erfindungsgemäß vorteilhaften Einsatzkonzentrationen für die UV-A-Filter (4-(tert.-Butyl)-4'-methoxydibenzoylmethan und 2-(4'-Diethylamino-2'-hydoxybenzoyl)-benzoesäurehexylester, Gesamtmenge) betragen von 1 bis 5 Gewichts-%, bevorzugt 1,5 bis 4,5 Gewichts-%, besonders bevorzugt 2 bis 4 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäüre-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Dabei ist es erfindungsgemäß bevorzugt, wenn die Zubereitung 2-Phenylbenzimidazol-5-sulfonsäure und/oder deren Salze enthält.
Enthält die erfindungsgemäße Zusammensetzung 2-Phenylbenzimidazol-5-sulfonsäure und/oder deren Salze, so ist es erfindungsgemäß vorteilhaft, wenn der Gehalt an diesem Stoff von 1 bis 5 Gewichts-%, bevorzugt 1,5 bis 4,5 Gewichts-%, besonders bevorzugt 2 bis 4 Gewichts-%, bezogen auf das Gesamtgewicht der Zubereitung beträgt.

Erfindungsgemäß bevorzugte Ausführungsformen sind auch dadurch gekennzeichnet, dass die Zubereitung 2-Ethylhexyl-2-cyano-3,3-diphenylacrylat enthält.

Es ist erfindungsgemäß vorteilhaft, wenn der Gehalt an Ethylhexylsalicylat in der Zubereitung von 1 bis 5 Gewichts-%, bevorzugt 1,5 bis 4,5 Gewichts-%, besonders bevorzugt 2 bis 4 Gewichts-% bezogen auf die Gesamtzubereitung beträgt.

Es ist erfindungsgemäß vorteilhaft, wenn der Gehalt an 2-Ethylhexyl-2-cyano-3,3-diphenylacrylat (Octocrylen) in der Zubereitung von 1 bis 10 Gewichts-%, bevorzugt 1,5 bis 5 Gewichts-%, besonders bevorzugt 2 bis 4 Gewichts-% bezogen auf die Gesamtzubereitung beträgt.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9), Talkum, Lauroyl Lysine und Acrylonitrilemethacrylonitrile-methyl-methacrylate.

Erfindungsgemäß bevorzugt ist es, wenn die Zubereitung Füllstoffe, insbesondere Polyethylen, Nylon, natürliche oder modifizierte Stärken wie Tapiokastärke und/oder Silikate wie beispielsweise Talkum enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl-oder-monobutylether, Propylenglykolmonomethyl, -monoethyl- oder-monobutylether, Diethylenglykolmonomethyl- oder -monoethylether und analoge Produkte, Schaumstabilisatoren, Elektrolyte, etc.. Erfindungsgemäß bevorzugt ist es, wenn die erfindungsgemäße Zubereitung dadurch gekennzeichnet ist, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält. Dabei werden diese Diole/Glycole erfindungsgemäß vorteilhaft in einer Gesamtmenge von 0,1 bis 3 Gewichts-%, bezogen auf das Gesamtgewicht der Zubereitung, eingesetzt.

Es ist erfindungsgemäß bevorzugt, wenn die Zubereitung Xanthangummi enthält.

Die ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlärge von 8 bis 24, insbesondere 12 bis 18 C-Atomen Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkelmöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bel der Fa. CP Hall erhältliche), Tridecylsalicylat (welches unter der Handelsbezeichnung Cosmaco. ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Nicht zuletzt ist es erfindungsgemäß vorteilhaft, wenn die Zubereitung Phenoxyethanol und/oder Methylisothiazolinon enthält.

### Vergleichsversuche

Mit den folgenden Versuchen konnte der erfindungsgemäße Effekt beispielhaft belegt werden:
Es wurde Beispiel 8 (Seite 10) des Dokumentes DE 20 2006 011 833 hergestellt (Rezeptur 1) und verglichen mit einer analogen Rezeptur 2, die zusätzlich 0,5 Gewichts-% Natriumpolyacrylat Homopolymer (INCI Sodium Polyacrylate) statt Wasser enthielt.

Beim Vergleich beider Zubereitungen zeigte sich, dass die Rezeptur 2 eine höhere Viskosität aufwies, beim Auftragen auf die Haut matter, d.h. weniger glänzend wirkte und sich weniger ölig anfühlte. Durch ihre, im "frischen" Zustand aufgetragenen, leicht gelblichen Schimmer, ließ sich die gleichmäßige Verteilung auf der Haut deutlich besser verfolgen als bei der Rezeptur 1. Die Röllchenbildung beim Verteilen der Zubereitungen auf der Haut, ist bei der Rezeptur 2 geringer als bei Rezeptur 1. Darüber hinaus lässt sich Rezeptur 2 leichter aus einem Handtuch wieder auswaschen als Rezeptur 1.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Rezepturbeispiele** | |
|---|---|
| **INCI** | **Sonnenemulsion** |
| Cetyl Alcohol | **1** |
| C12-15 Alkyl Benzoate | **3** |
| Cyclomethicone | **2** |
| Polyglyceryl-3 Methylglucose Distearate | **2** |
| Distarch Phosphate | **2** |
| Parfum | **0,3** |
| Glycerin | **2,6** |
| Aqua + Sodium Hydroxide | **1** |
| Phenoxyethanol | **0,8** |
| Methylisothiazolinone | **0,09** |
| Carbomer | **0,4** |
| Sodium Polyacrylate | **0,1** |
| Alcohol Denat. | **2** |
| Aqua + Trisodium EDTA | **1** |
| Octocrylene | **2,5** |
| Butyl Methoxydibenzoylmethane | **2** |
| Phenylbenzimidazole Sulfonic Acid | **2** |
| Ethylhexyl Salicylate | **4,5** |
| Aqua | **ad 100** |

## Patentansprüche

1. Kosmetische Emulsion enthaltend
a) Polyglyceryl-3 Methylglucose Distearate,
b) Natriumpolyacrylat Homopolymer (INCI Sodium Polyacrylate) und
c) quervernetzte Polyacrylsäure und/oder deren Salze, **dadurch gekennzeichnet, dass** die Zubereitung Ethylhexylsalicylat enthält,
wobei das Gewichtsverhältnis von Natriumpolyacrylat Homopolymer (INCI Sodiumpolyacrylate) zu quervernetzter Polyacrylsäure und/oder deren Salze von 1:7 bis 1:2 beträgt.

2. Kosmetische Emulsion nach Anspruch 1, **dadurch gekennzeichnet, dass** die Zubereitung einen oder mehrere UV-A-Filter gewählt aus der Gruppe der Verbindungen 4-(tert.-Butyl)-4'-methoxydibenzoylmethan und 2-(4'-Diethylamino-2'-hydoxybenzoyl)-benzoesäurehexylester enthält.

3. Kosmetische Zubereitung nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

4. Kosmetische Zubereitung nach einem der vorhergehenden Ansprüche, **dadurch gekennzeichnet, dass** die Zubereitung 2-Phenylbenzimidazol-5-sulfonsäure und/oder deren Salze enthält.

5. Kosmetische Zubereitung nach einem der vorhergehenden Ansprüche, **dadurch gekennzeichnet, dass** die Zubereitung 2-Ethylhexyl-2-cyano-3,3-diphenylacrylat enthält.

6. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische isoflavonoide, Glycerylglucose, Kreatin, Kreatinin, Taurin, Tocopherol, Tocopherolacetat, ß-Alanin und/oder Licochalcon A, enthält.

7. Kosmetische Zubereitung nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

8. Kosmetische Zubereitung nach einem der vorhergehenden Ansprüche, **dadurch gekennzeichnet, dass** die Zubereitung Phenoxyethanol und/oder Methylisothiazolinon enthält.

9. Kosmetische Zubereitung nach einem der vorhergehenden Ansprüche, **dadurch gekennzeichnet, dass** die Zubereitung Polyglyceryl-3 Methylglucose Distearate in einer Konzentration von 0,5 bis 4 Gewichts-%, bezogen auf das Gesamtgewicht der Zubereitung, enthält.

10. Kosmetische Zubereitung nach einem der vorhergehenden Ansprüche, **dadurch gekennzeichnet, dass** die Zubereitung Natriumpolyacrylat Homopolymer (INCI Sodium Polyacrylate) in einer Konzentration von 0,01 bis 1 Gewichts-%, bezogen auf das Gesamtgewicht der Zubereitung, enthält.

11. Kosmetische Zubereitung nach einem der vorhergehenden Ansprüche, **dadurch gekennzeichnet, dass** die Zubereitung quervernetzte Polyacrylsäure und/oder deren Salze in einer Konzentration von 0,1 bis 1 Gewichts-%, bezogen auf das Gesamtgewicht der Zubereitung, enthält.

## Claims

1. Cosmetic emulsion comprising
a) polyglyceryl-3 methylglucose distearate
b) sodium polyacrylate homopolymer (INCI Sodium Polyacrylate) and
c) crosslinked polyacrylic acid and/or salts thereof, **characterized in that** the preparation comprises ethylhexyl salicylate, wherein the ratio by weight of sodium polyacrylate homopolymer (INCI: Sodium Polyacrylate) to crosslinked polyacrylic acid and/or salts thereof is from 1:7 to 1:2.

2. Cosmetic emulsion according to Claim 1, **characterized in that** the preparation comprises one or more UVA filters selected from the group of compounds comprising 4-(tert-butyl)-4'-methoxydibenzoylmethane and hexyl 2-(4'-diethylamino-2'-hydroxybenzoyl)benzoate.

3. Cosmetic preparation according to either of the preceding claims, **characterized in that** the preparation comprises one or more UV filters selected from the group of compounds comprising 2-phenylbenzimidazole-5-sulphonic acid and/or salts thereof; phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4- (1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; terephthalidenedicamphorsulphonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bisethoxycarbonylvinyl)phenoxy)propenyl)methoxysiloxane/dimethylsiloxan e copolymer; dioctylbutylamidotriazone (INCI: Diethylhexyl Butamido Triazone); 2,4-bis[5-1 (dimethylpropyl)benzoxazol-2-yl(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

4. Cosmetic preparation according to any of the preceding claims, **characterized in that** the preparation comprises 2-phenylbenzmidazole-5-sulphonic acid and/or salts thereof.

5. Cosmetic preparation according to any of the preceding claims, **characterized in that** the preparation comprises 2-ethylhexyl 2-cyano-3,3-diphenylacrylate.

6. Cosmetic preparation according to any of the preceding claims, **characterized in that** the preparation comprises one or more active ingredients selected from the group of compounds comprising glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

7. Cosmetic preparation according to any of the preceding claims, **characterized in that** the preparation comprises propylene glycol, butylene glycol, 2-methylpropane-1,3-diol, 1,2-pentanediol, 1,2-hexanediol, 1,2-octanediol and/or 1,2-decanediol.

8. Cosmetic preparation according to any of the preceding claims, **characterized in that** the preparation comprises phenoxyethanol and/or methylisothiazolinone.

9. Cosmetic preparation according to any of the preceding claims, **characterized in that** the preparation comprises polyglyceryl-3 methylglucose distearate at a concentration of 0.5 to 4% by weight, based on the total weight of the preparation.

10. Cosmetic preparation according to any of the preceding claims, **characterized in that** the preparation comprises sodium polyacrylate homopolymer (INCI: Sodium Polyacrylate) at a concentration of 0.01 to 1% by weight, based on the total weight of the preparation.

11. Cosmetic preparation according to any of the preceding claims, **characterized in that** the preparation comprises crosslinked polyacrylic acid and/or salts thereof at a concentration of 0.1 to 1% by weight, based on the total weight of the preparation.

## Revendications

1. Emulsion cosmétique contenant
a) du polyglycéryl-3 méthylglucose distéarate,
b) un homopolymère de polyacrylate de sodium (INCI Sodium Polyacrylate) et
c) du poly(acide acrylique) réticulé et/ou ses sels, **caractérisée en ce que** la préparation contient du salicylate d'éthylhexyle, le rapport pondéral de l'homopolymère de polyacrylate de sodium (INCI Sodium Polyacrylate) au poly(acide acrylique) réticulé et/ou ses sels étant de 1:7 à 1:2.

2. Emulsion cosmétique selon la revendication 1, **caractérisée en ce que** la préparation contient un ou plusieurs filtres UV-A, choisis dans le groupe formé par les composés 4-(tert-butyl)-4'-méthoxydibenzoylméthane et ester hexylique de l'acide 2-(4'-diéthylamino-2'-hydroxybenzoyl)-benzoïque.

3. Préparation cosmétique selon l'une quelconque des revendications précédentes, **caractérisée en ce que** la préparation contient un ou plusieurs filtres UV, choisis dans le groupe des composés ; acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; acide téréphtalidènedicamphresulfonique ; 2-cyano-3,3-diphénylacrylate de 2-éthylhexyle ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2-dihydroxy-4-méthoxybenzophénone, salicylate d'homomenthyle ; 2-hydroxybenzoate de 2-éthylhexyle ; benzalmalonate de diméthicodiéthyle ; copolymère de 3-(4-(2,2-bis éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (présentant le n° CAS 288254-16-0) ; ester tris-(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc.

4. Préparation cosmétique selon l'une quelconque des revendications précédentes, **caractérisée en ce que** la préparation contient de l'acide 2-phénylbenzimidazole-5-sulfonique et/ou ses sels.

5. Préparation cosmétique selon l'une quelconque des revendications précédentes, **caractérisée en ce que** la préparation contient du 2-cyano-3,3-diphénylacrylate de 2-éthylhexyle.

6. Préparation cosmétique selon l'une quelconque des revendications précédentes, **caractérisée en ce que** la préparation contient une ou plusieurs substances actives choisies dans le groupe des composés acide glycyrrhétinique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

7. Préparation cosmétique selon l'une quelconque des revendications précédentes, **caractérisée en ce que** la préparation contient du propylèneglycol, du butylèneglycol, du 2-méthylpropane-1,3-diol, du 1,2-pentanediol, du 1,2-hexanediol, du 1,2-octanediol et/ou du 1,2-décanediol.

8. Préparation cosmétique selon l'une quelconque des revendications précédentes, **caractérisée en ce que** la préparation contient du phénoxyéthanol et/ou de la méthylisothiazolinone.

9. Préparation cosmétique selon l'une quelconque des revendications précédentes, **caractérisée en ce que** la préparation contient du polyglycéryl-3 méthylglucose distéarate en une concentration de 0,5 à 4% en poids par rapport au poids total de la préparation.

10. Préparation cosmétique selon l'une quelconque des revendications précédentes, **caractérisée en ce que** la préparation contient de l'homopolymère de polyacrylate de sodium (INCI Sodium polyacrylate) en une concentration de 0,01 à 1% en poids, par rapport au poids total de la préparation.

11. Préparation cosmétique selon l'une quelconque des revendications précédentes, **caractérisée en ce que** la préparation contient du poly(acide acrylique) réticulé et/ou ses sels en une concentration de 0,1 à 1% en poids par rapport au poids total de la préparation.
